# EUROPÄISCHE PATENTSCHRIFT

(11) **EP 1 244 518 B1**
(45) Veröffentlichungstag und Bekanntmachung des Hinweises auf die Patenterteilung: **30.07.2003**
(21) Anmeldenummer: 00990602.5
(22) Anmeldetag: 16.11.2000
(51) Int. Cl.: B01J 27/02

(54) **OLIGOMERISIERUNGSKATALYSATOR, VERFAHREN ZU SEINER HERSTELLUNG UND SEINE VERWENDUNG**
OLIGOMERISATION CATALYST, A METHOD FOR PRODUCTION AND THE USE THEREOF
CATALYSEUR D'OLIGOMERISATION, SON PROCEDE DE PREPARATION ET SON UTILISATION

(30) Priorität: 27.11.1999 DE 19957173
(43) Veröffentlichungstag der Anmeldung: 02.10.2002
(73) Patentinhaber: BASF AKTIENGESELLSCHAFT, 67056 Ludwigshafen (DE)
(72) Erfinder: WALTER, Marc, 67227 Frankenthal (DE); HEIDEMANN, Thomas, 69469 Weinheim (DE)
(86) Internationale Anmeldenummer: EP0011344
(87) Internationale Veröffentlichungsnummer: WO01037989

(56) Entgegenhaltungen:
- EP-A- 0 272 970
- FR-A- 2 641 477
- US-A- 2 794 842
- US-A- 3 959 400

## Beschreibung

Die vorliegende Erfindung betrifft ein Verfahren zur Herstellung eines Oligomerisierungskatalysators für Olefine mit 4 bis 6 Kohlenstoffatomen, bei dem man Aluminiumoxid mit einer Nickelverbindung und einer Schwefelverbindung beaufschlagt, und zwar gleichzeitig oder zuerst mit der Nickelverbindung und danach mit der Schwefelverbindung, und den so erhaltenen Katalysator anschließend trocknet und calciniert.

Weiterhin betrifft die Erfindung die so hergestellten Katalysatoren und ein Verfahren zur Herstellung von Oligomeren von Olefinen mit 4 bis 6 Kohlenstoffatomen oder von Gemischen dieser Olefine.

Olefine mit 4 bis 6 Kohlenstoffatomen und deren Gemische stehen in großen Mengen sowohl aus FCC-Anlagen als auch aus Steamcrackern zur Verfügung. Der jeweilige C₄-Schnitt, d.h. das Gemisch aus Butenen und Butanen eignet sich nach Abtrennung des Isobutens sehr gut zur Herstellung von Oligomeren, insbesondere Octenen und Dodecenen. Sowohl die Octene als auch Dodecene werden nach Hydroformylierung und nachfolgender Hydrierung zu den entsprechenden Alkoholen z.B. für die Herstellung von Weichmachern oder Tensidalkoholen verwendet.

Für den Einsatz als Weichmacheralkohol spielt der Verzweigungsgrad für die Eigenschaften des Weichmachers eine ausschlaggebende Rolle. Der Verzweigungsgrad wird durch den ISO-Index beschrieben, der die mittlere Zahl der Methylverzweigungen in der jeweiligen Fraktion angibt. So tragen z.B. n-Octene mit 0, Methylheptene mit 1 und Dimethylhexene mit 2 zum ISO-Index einer Fraktion bei. Je niedriger der ISO-Index ist, um so linearer sind die Moleküle in der jeweiligen Fraktion aufgebaut. Je höher die Linearität, d.h. je niedriger der ISO-Index ist, um so höher sind die Ausbeuten in der Oxierung und umso besser sind die Eigenschaften des damit hergestellten Weichmachers. Ein niedriger ISO-Index wirkt sich etwa bei Weichmachern dahingehend aus, dass sie weniger flüchtig sind, und PVC zeigt ein verbessertes Bruchverhalten bei Kälte, wenn es solche Weichmacher enthält.

Die Oligomerisierung wird großtechnisch entweder homogen- oder heterogenkatalytisch ausgeführt.

Bei der heterogen-katalytischen Arbeitsweise kennt der Stand der Technik bereits nickel- und schwefelhaltige Aluminiumoxid-Katalysatoren. Die JP-B 74/3489 (Nippon Oil Co. Ltd.) beschreibt die Verwendung von Nickelsulfid auf Aluminiumoxid als Katalysator für die Herstellung von niederen Olefinpolymeren.

In vielen Fällen werden Nickel und Sulfat in Form von Nickelsulfat auf den Träger aufgebracht, wodurch sich im fertigen Katalysator normalerweise Ni:S-Verhältnisse um den Wert 1 ergeben: Der US-A 2 794 842 (Phillips Petroleum Co.) kann man die Polymerisation von Olefinen unter Verwendung von Nickelsulfat auf einem Aluminiumoxid-Träger entnehmen. In der US-A 3 959 400 (Mobil Oil Corp.) wird ein Verfahren vorgestellt, bei dem die katalytische Dimerisierung von Olefinen mit 2 bis 4 Kohlenstoffatomen an Aluminiumoxiden erfolgt, die mit Nickelsulfat belegt wurden. Die FR-A 2 641 477 (Institut Francais du Petrole) lehrt die Dimerisierung von Olefinen anhand eines Aluminiumoxids, dass mit Nikkelsulfat beaufschlagt wurde. Die US-A 4 511 750 (Chevron Research Co.) beschreibt die Oligomerisierung niederer Olefine mittels Nickelsulfat auf einem porösen anorganischen Oxid, beispielsweise Aluminiumoxid.

Aus der JP-A 73/85506 (Koa Sekiyu K.K.) ist ein Verfahren zur Herstellung von Octenen bekannt, bei dem man einen Katalysator auf Basis von Nickeloxid/Siliciumdioxid, Nickeloxid/Aluminiumoxid oder Nickeloxid/Siliciumdioxid/Aluminiumoxid einsetzt, der mit einem Metallsalz versetzt wurde. Das Metallsalz kann dabei ein Sulfat sein.

Katalysatoren, deren Ni:S-Verhältnisse vom Wert 1 abweichen, sind ebenfalls bekannt:

Die US-A 5 883 036 (Koa Oil Co. Ltd.) und die JP-A 98/101586 lehren die Herstellung eines mit Sulfationen und Nickeloxid beaufschlagten Aluminiumoxids als Katalysators für die Olefinoligomerisierung. Die Belegung mit den Sulfationen erfolgt vor der Belegung mit dem Nickeloxid.

Aus der EP-A 272 970 (Institut Francais du Petrole) ist ein Verfahren zur Dimerisierung monoolefinischer Kohlenwasserstoffe bekannt, bei dem ein Aluminiumoxid mit einem bestimmten Gehalt an Sulfationen und mit von 0,5 bis 15 Gew.-% Nickel verwendet wird. Darüberhinaus liegen die molaren Verhältnisse von Schwefel und Nickel in dem Katalysator bei 0,1 : 1 bis 0,95 : 1. Bei der Herstellung dieses Katalysators lässt man entweder die Sulfationenquelle und die Nickelionenquelle gleichzeitig auf das Aluminiumoxid einwirken, trocknet anschließend und calciniert. Alternativ belegt man das Aluminiumoxid mit einem wärmezersetzlichen Nickelsalz, trocknet, calciniert und bringt danach die Sulfationen auf.

Die Dimerenselektivität und der Alkylketten-Verzweigungsgrad der mit den bekannten Katalysatoren auf Basis Schwefel- und Nickelhaltiger Aluminiumoxide hergestellten Olefinoligomeren kann jedoch noch nicht befriedigen.

Der vorliegenden Erfindung lagen daher derartige Katalysatoren als Aufgabe zugrunde, mit denen bei guten Olefinumsätzen höhere Dimerenselektivität und höhere Grade der Linearität der Alkylgruppen erreicht werden können.

Diese Aufgabe wurde erfindungsgemäß gelöst durch ein Verfahren zur Herstellung eines Oligomerisierungskatalysators für Olefine mit 4 bis 6 Kohlenstoffatomen, bei dem man Aluminiumoxid mit einer Nickelverbindung und einer Schwefelverbindung beaufschlagt, und zwar gleichzeitig oder zuerst mit der Nickelverbindung und danach mit der Schwefelverbindung, und den so erhaltenen Katalysator anschließend trocknet und calciniert, wobei man auf diese Weise im fertigen Katalysator ein molares Verhältnis von Schwefel zu Nickel von 0,25 : 1 bis 0,38 : 1 einstellt.

Daneben wurden die derart erhältlichen neuen Katalysatoren und ein Verfahren zur Herstellung von Oligomeren von Olefinen mit 4 bis 6 Kohlenstoffatomen oder von Gemischen dieser Olefine gemäß Anspruch 8 gefunden.

Unter "Oligomeren" werden hierin Dimere, Trimere und höhere Oligomere der Olefine mit 4 bis 6 Kohlenstoffatomen verstanden. Das erfindungsgemäße Verfahren eignet sich insbesondere zur Herstellung von Dimeren dieser Olefine.

Der erfindungsgemäße Katalysator kann auf verschiedenen Wegen hergestellt werden, beispielsweise durch
a) Co-Fällung aus Lösungen, die die Katalysator-Bestandteile in gelöster Form enthalten: der Niederschlag wird gewaschen, getrocknet, verformt und calciniert (vgl. dazu etwa DE-A 43 39 713), oder
b) durch Tränken des Aluminiumoxids, das als Pulver oder bereits verformt vorliegt, mit einer Lösung der Nickel- und der Schwefelverbindung und anschließendes Trocknen und Calcinieren des erhaltenen Produkts.

Man stellt die erfindungsgemäßen Katalysatoren vorzugsweise nach der Variante b) her und zwar unter Verwendung von vorgefertigten Aluminiumoxid-Formkörpern, beispielsweise Kugeln und vor allem Stränge oder Sternenstränge mit einem größten Durchmesser von 1 bis 3 mm. Die vorstehende Größenangabe ist lediglich beispielhaft und stellt keine Einschränkung des Gegenstandes der vorliegenden Erfindung dar. In stückiger Form erhält man den Aluminiumoxid-Träger, indem man den Aluminiumoxid-Vorläufer oder das aus diesem durch Calcinieren erhaltene Pulver den jeweiligen Anforderungen entsprechend in an sich bekannter Weise verformt (vgl. Handbook of Heterogenous Catalysis, Vol. 1, Seite 80 ff.).

Die chemischen und physikalischen Eigenschaften der bei der Katalysatorherstellung verwendeten Aluminiumoxide hängen bekanntermaßen stark von deren Herkunft ab. Sie sind in sich bekannter Weise zugänglich als auch kommerziell erhältlich. Man erhält sie etwa durch Calcinieren von Aluminiumoxid-Vorläufern wie Böhmit und Hydrargillit.

Für die Herstellung der erfindungsgemäßen Katalysatoren verwendet man vorzugsweise γ-Aluminiumoxid, η-Aluminiumoxid sowie deren Gemische, wie sie etwa von den Firmen BASF, Condea, Alcoa, Grace und Rhone-Poulenc kommerziell erhältlich sind. Bevorzugt sind Aluminiumoxid-Träger, die überwiegend und vor allem vollständig aus γ-Aluminiumoxid bestehen. Vorzugsweise haben diese Aluminiumoxide ein Wasseraufnahmevermögen von mehr als 0,4 ml/g und eine BET-Oberfläche von mehr als 150 m²/g. Weiterhin bevorzugt werden derartige Aluminiumoxide mit einem Gehalt von weniger als 0,2 Gew.-% Na₂O eingesetzt. Außerdem bevorzugt werden derartige Aluminiumoxide mit einem Gehalt von weniger als 0,2 Gew.-% Fe₂O₃ eingesetzt.

Das Aluminiumoxid beaufschlagt man mit der Nickelverbindung und der Schwefelverbindung besonders zweckmäßig durch Tränken mit den Lösungen dieser Verbindungen.

Beim Tränken setzt man die Schwefelverbindung und die Nickelverbindung vorzugsweise als deren Lösung insbesondere in Wasser und nachgeordnet in organischen polaren Lösungsmittel, z.B. Alkoholen wie Methanol und Ethanol, oder Gemischen der geeigneten Lösungsmittel ein.

Als Schwefelverbindung eignen sich Sulfate und alle solche Verbindungen des Schwefels, die sich beim Erhitzen in Gegenwart von Sauerstoff oder von Sauerstoff enthaltenden Gasgemischen wie Luft unter den Calcinierungsbedingungen in Sulfat überführen lassen, beispielsweise die Sulfide. Vorzugsweise verwendet man wasserlös-liche Sulfate wie Ammoniumsulfat, das sich bei ca. 250°C zersetzt und vor allem Schwefelsäure.

Als Nickelverbindung eignen sich alle Verbindungen des Nickels, die sich beim Erhitzen in Gegenwart von Sauerstoff oder von Sauerstoff enthaltenden Gasgemischen wie Luft unter den Calcinierungsbedingungen in eine oxidische Form des Metalls überführen lassen. Vorzugsweise verwendet man als Nickelverbindung wasserlösliche Nickelsalze, beispielsweise mit organischem Anion wie das Formiat, Oxalat, Acetylacetonat oder das 2-Ethylhexanoat und vor allem gegebenenfalls hydratisiertes Nickelnitrat.

Beim Tränken verrührt man das Aluminiumoxid vorzugsweise mit einer Lösung, die genau diejenigen Stoffmengen an Nickelverbindung und Schwefelverbindung enthält, mit denen das Aluminiumoxid beaufschlagt werden soll. Dabei wählt man das Volumen der Lösung vorteilhafterweise so, dass es gerade dem Wasseraufnahmevermögen des Aluminiumoxids entspricht.

Alternativ können die Nickel- und die Schwefelverbindung auch durch mehrere Tränkschritte aufgebracht werden, nach denen die erhaltene Katalysator-Vorstufe jeweils getrocknet wird.

Wird die Schwefelverbindung erfindungsgemäß erst nach der Nickelverbindung auf das Aluminiumoxid aufgebracht, so arbeitet man vorzugsweise so, dass man das mit der Nickelverbindung beaufschlagte Aluminiumoxid zuvor bei 50 bis 200°C trocknet.

Im übrigen verfährt man beim Tränken des Aluminiumoxids in an sich bekannter Weise (vgl. hierzu etwa die EP-B 272 970).

Der getränkte Katalysator wird anschließend getrocknet, und zwar vorzugsweise unter Luft und bei 50 bis 200°C.

Das Calcinieren wird in einer sauerstoffhaltigen Atmosphäre, vorzugsweise unter Luft durchgeführt. Die Temperatur für das Calcinieren liegt normalerweise im Bereich von 300 bis 600°C. Beim Calcinieren wird aus der Nickelverbindung und der Schwefelverbindung die katalytisch wirksame oxidische Nickel- und Schwefel-haltige Aktivmasse gebildet.

Läßt man entgegen der Erfindung erst die Schwefelverbindung und dann die Nickelverbindung auf das Aluminiumoxid einwirken, so erhält man bei der Oligomerisierung unerwünscht hohe ISO-Indizes.

Die auf diese Weise hergestellten neuen Katalysatoren werden vor ihrer Verwendung bei der Oligomerisierung zweckmäßigerweise einer Konditionierung im trockenen Stickstoffstrom, z.B. bei Atmosphärendruck und Temperaturen von 20 bis 500°C, vorzugsweise 100 bis 250°C unterworfen, um Feuchtigkeitsspuren (etwa aus der Luft) aus dem Katalysator zu entfernen.

Das mittels Elementaranalyse ermittelte molare Verhältnis von Schwefel und Nickel im erfindungsgemäßen Katalysator beträgt vorzugsweise 0,28 : 1 bis 0,35 : 1. Oberhalb dieses Bereiches nimmt normalerweise die Dimerenselektivität, darunter in der Regel die Katalysatoraktivität deutlich ab.

Das erfindungsgemäße Oligomerisierungverfahren eignet sich besonders für Gemische von Olefinen mit 4 Kohlenstoffatomen. Vor allem eignet es sich für die Umsetzung von Gemischen, die Olefine mit 4 Kohlenstoffatomen enthalten, insbesondere Kohlenwasserstoffströme, die 1-Buten und/oder 2-Buten und Butan enthalten und die im Wesentlichen von Isobuten frei sind, zu Dodecenen und insbesondere zu Octenen. Geeignete Kohlenwasserströme mit Olefinen mit 4 Kohlenstoffatomen sind z.B. Gemische mit folgender Zusammensetzung:

| | |
|---|---|
| Butane | 10 bis 90 Gew.-% |
| Butene | 10 bis 90 Gew.-%, |

wobei die Buten-Fraktion folgende Zusammensetzung haben kann:

| | |
|---|---|
| 1-Buten | 1 bis 50 Gew.-% |
| cis-2-Buten | 1 bis 50 Gew.-% |
| trans-2-Buten | 1 bis 99 Gew.-% |
| iso-Buten | 1 bis 5 Gew.-% |

Als besonders bevorzugter Einsatzstoff wird das sogenannte Raffinat II verwendet. Es handelt sich dabei um ein Buten-haltiges C₄-Kohlenwasserstoffgemisch, wie es aus dem C₄-Schnitt von Crackern erhalten wird, nachdem höher ungesättigte Kohlenwasserstoffe, wie Diolefine, insbesondere 1,3-Butadien, oder Acetylen und danach das enthaltene Isobuten abgetrennt worden sind. Eine typische Zusammensetzung für Raffinat II ist z.B.:

| | |
|---|---|
| iso-, n-Butan | 26 Gew.-% |
| iso-Buten | 1 Gew.-% |
| 1-Buten | 26 Gew.-% |
| trans-2-Buten | 31 Gew.-% |
| cis-2-Buten | 16 Gew.-% |

Die C₄-Kohlenwasserstoffströme können z.B. in an sich etwa aus der DE-A 39 14 817 bekannten Weise von Butadien, schwefelhaltigen und sauerstoffhaltigen Verbindungen wie Alkoholen, Aldehyden, Ketonen oder Ethern, durch selektive Hydrierung bzw. Absorption an einem Molekularsieb befreit werden.

Die Oligomerisierungsreaktion findet vorzugsweise von 30 bis 140°C und insbesondere von 30 bis 120°C und einem Druck vorzugsweise von 15 bis 100 bar und insbesondere von 20 bis 70 bar statt. Der Druck wird dabei zweckmäßig so ausgewählt, dass bei der eingestellten Temperatur das Einsatzkohlenwasserstoffgemisch flüssig oder im überkritischen Zustand vorliegt. Der Reaktor ist in der Regel ein mit dem Katalysator beschickter zylindrischer Reaktor, der von dem flüssigen Reaktionsgemisch z.B. von oben nach unten durchströmt wird. Das erfindungsgemäße Oligomerisierungsverfahren kann in einem einzelnen Reaktor bis zum gewünschten Endumsatz der Butene durchgeführt werden, wobei der Oligomerisierungskatalysator in einem einzigen oder mehreren Festbetten im Reaktor angeordnet sein kann. Alternativ kann zur Durchführung des erfindungsgemäßen Verfahrens eine Reaktorkaskade aus mehreren, vorzugsweise zwei, hintereinander geschalteten Reaktoren eingesetzt werden; beim Passieren des bzw. der dem letzten Reaktor der Kaskade vorgeschalteten Reaktors bzw. Reaktoren ist es möglich, die Oligomerisierung der Kohlenwasserstoffe im Reaktionsgemisch nur bis zu einem Teilumsatz zu betreiben und der gewünschte Endumsatz wird erst beim Passieren des Reaktionsgemisches durch den letzten Reaktor der Kaskade erzielt. In den einzelnen Reaktoren der Reaktorkaskade kann der Oligomerisierungskatalysator in einem einzigen oder in mehreren Katalysatorfestbetten angeordnet sein. Weiterhin können in den einzelnen Reaktoren der Reaktorkaskade unterschiedliche Reaktionsbedingungen hinsichtlich Druck und/oder Temperatur im Rahmen der obengenannten Druck- und Temperaturbereiche eingestellt werden. Außerdem ist es möglich, in den einzelnen Reaktoren der Kaskade unterschiedliche Oligomerisierungskatalysatoren einzusetzen, obgleich die Anwendung des gleichen Katalysators in sämtlichen Reaktoren der Kaskade bevorzugt wird.

Von einer einstufigen Reaktionszone wird gesprochen, wenn ein einziger Oligomerisierungsreaktor eingesetzt wird oder wenn eine Reaktorkaskade verwendet wird, in deren Reaktoren der gleiche Oligomerisierungskatalysator eingesetzt wird.

Werden in den einzelnen Reaktoren der Kaskade unterschiedliche Oligomerisierungskatalysatoren angewandt, wird dies als mehrstufige Reaktionszone bezeichnet.

Man kann man die Oligomerisierung unter überkritischen Bedingungen in bezug auf das Einsatzmaterial durchführen, wobei man vorzugsweise keine zusätzlichen Lösungsmittel verwendet, die sich nicht im überkritischen Zustand befinden.

Nach dem Verlassen der ein- oder mehrstufigen Reaktionszone werden die gebildeten Oligomere in an sich bekannter Weise von den nicht umgesetzten Kohlenwasserstoffen getrennt und diese Kohlenwasserstoffe vollständig oder zum größten Teil in an sich bekannter Weise zurückgeführt (vgl. etwa die WO-A 95/14647).

Das erfindungsgemäße Oligomerisierungsverfahren kann adiabatisch oder isotherm durchgeführt werden.

Im technischen Sinne wird unter einer adiabatischen Reaktionsführung oder Betriebsweise eine Reaktionsführung oder Betriebsweise verstanden, bei der, abgesehen von dem durch natürliche Wärmeleitung und Wärmeabstrahlung vom Reaktor an die Umgebung abgegebenen Teil der Reaktionswärme, die gesamte Reaktionswärme vom Reaktionsgemisch aufgenommen und mit diesem aus dem Reaktor abgeführt wird.

Bei der isothermen Reaktionsführung im technischen Sinne wird dagegen der Austrag der Reaktionswärme aus dem Reaktor mittels Kühl- oder Thermostatisiervorrichtungen über das durch natürliche Wärmeleitung oder Wärmeabstrahlung gegebene Maß hinaus gezielt forciert, wobei die Reaktionswärme in der Regel zunächst von einem Wärmeträgermedium, dem Kühlmittel, aufgenommen wird, bevor sie an die Umgebung abgegeben oder, beispielsweise bei Verwendung von Wärmetauschern, zur Erwärmung von Stoffen oder zur Energiegewinnung, genutzt wird (vgl. etwa die WO-A 95/14647).

Die Auftrennung der Oligomeren erfolgt in an sich bekannter Weise, vor allem durch fraktionierte Destillation, insbesondere zu den gewünschten Octen- und Dodecenfraktionen, die als Ausgangsmaterialien für die Hydroformylierung zur Herstellung der Weichmacheralkohole Nonanol und Tridecanol dienen. Aufgrund der geringen Verzweigung der Ausgangsoligomeren weisen die daraus hergestellten höheren Alkohole hervorragende Eigenschaften für die Herstellung von Weichmachern und Tensiden auf.

### Beispiele

### I. Katalysatoren

Für die Herstellung der Katalysatoren kamen die in Tabelle 1 zusammengestellten γ-Aluminiumoxid-Träger zum Einsatz.

Es wurde γ-Aluminiumoxid vom Typ "D10-10" der Firma BASF oder vom Typ "Pural" der Firma Condea verwendet.

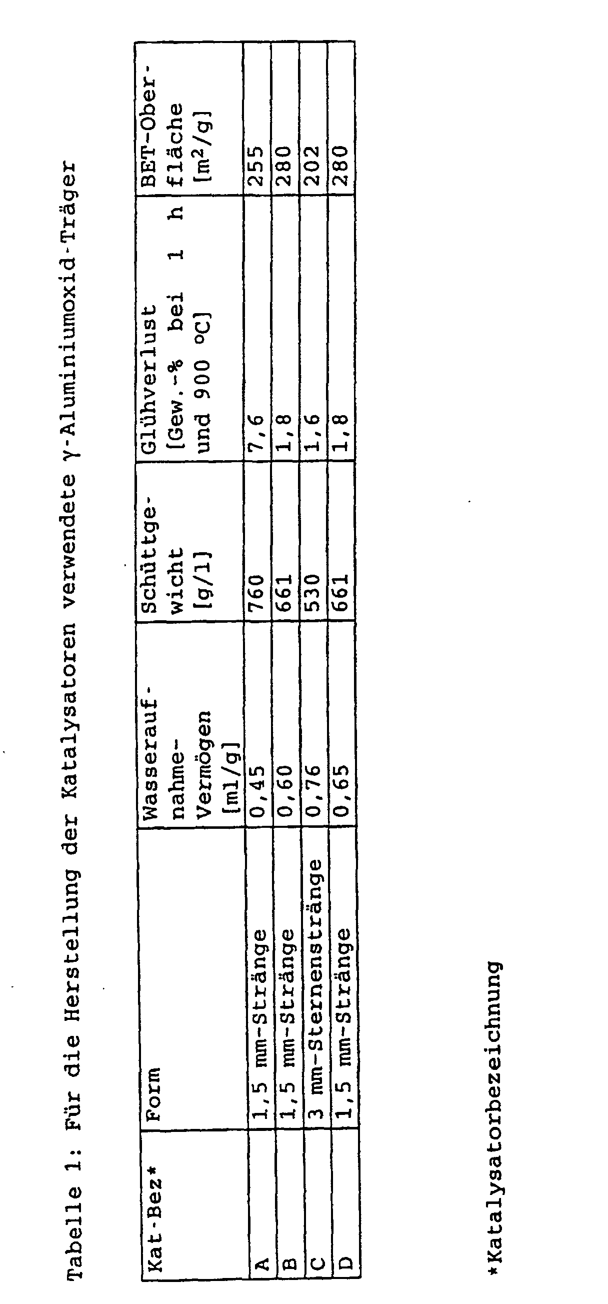

### Allgemeine Herstellvorschrift für die Katalysatoren (die zugehörigen Zahlenwerte finden sich in der anschließenden Tabelle 2)

Das verwendete Ni(NO₃)₂·6 H₂O stammte von der Firma Fluka.

Zur Herstellung der Katalysatoren wurde der jeweilige Träger bei Raumtemperatur mit einer Lösung aus den unten angegebenen Mengen von 96 %-iger H₂SO₄, 97 %-igem Ni(NO₃)₂ · 6 H₂O und Wasser unter Rühren getränkt. Der so erhaltene Katalysator wurde 10 Stunden bei 120°C unter Luft getrocknet und 2 Stunden unter Luft bei 500°C calciniert. Danach wurden die prozentualen Anteile des Nickels ("Ni") und des Schwefels ("S") am Gesamtgewicht des erhaltenen Katalysators sowie das molare Verhältnis von Schwefel zu Nickel ("S:Ni") im Katalysator ermittelt.

Der Gehalt des fertigen Katalysators an Schwefel wurde über die quantitative Infrarot-Analyse des bei der Verbrennung des Katalysators entstandenen Schwefeldioxids ermittelt. Der Gehalt an Nikkel war über ICP-Massenspektrometrie zugänglich.

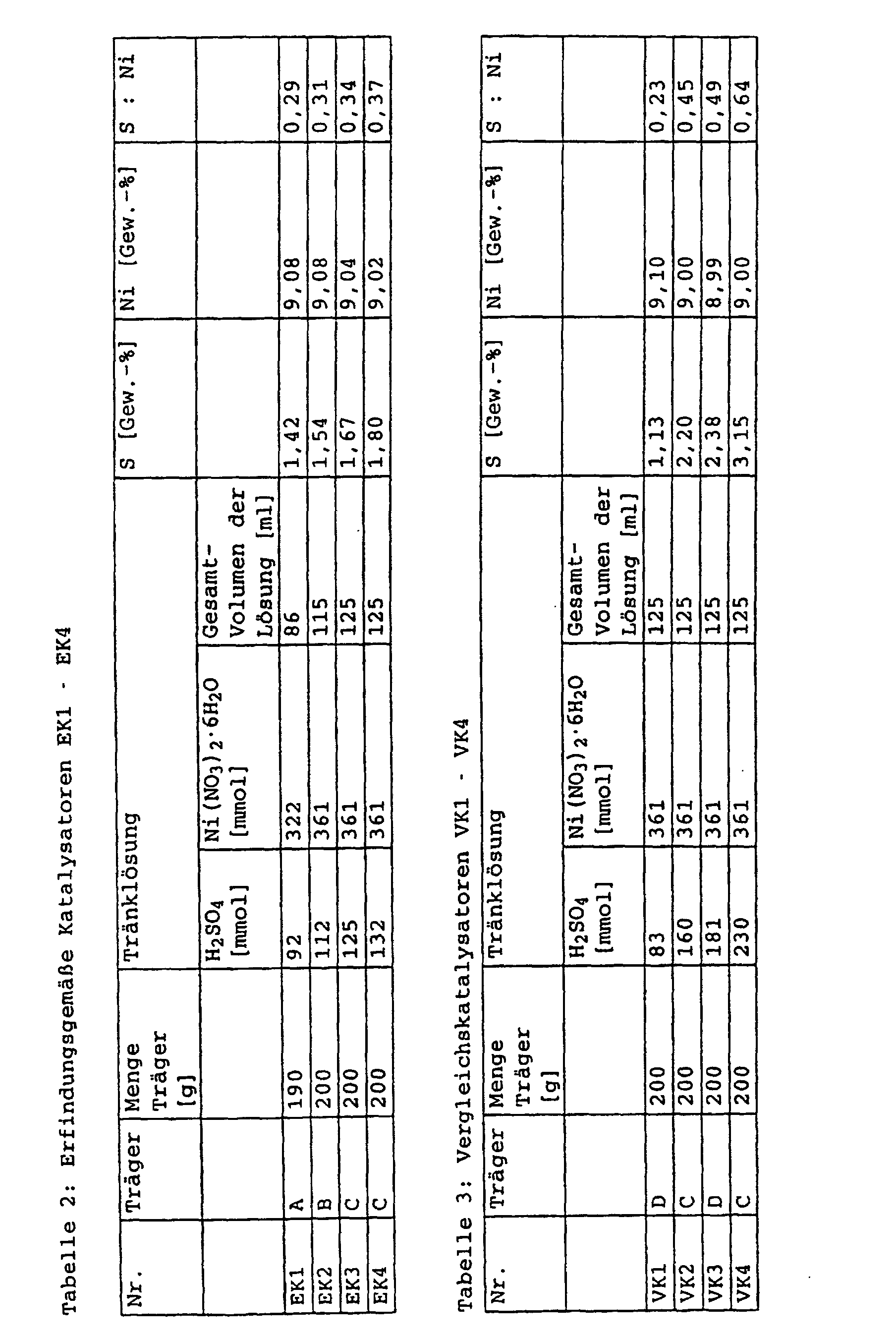

### Vergleichskatalysator VK5 (Sulfat vor Nickel aufgebracht)

380 g Träger A wurden bei Raumtemperatur mit 171 ml einer Lösung aus 184 mmol H₂SO₄ (96 %-ig) und Wasser unter Rühren getränkt. Das Produkt wurde 3 Stunden bei 120°C unter Luft getrocknet und 2 Stunden bei 500°C unter Luft calciniert. Der so erhaltene Katalysator wurde anschließend bei Raumtemperatur mit 171 ml einer Lösung aus 644 mmol Ni(NO₃)₂·6 H₂O (97 %-ig) und Wasser unter Rühren getränkt. Danach wurde der so erhaltene Katalysator 2 Stunden bei 120°C unter Luft getrocknet und 2 Stunden bei 500°C unter Luft calciniert. Anschließend wurde der prozentuale Anteil des Nickels zu 9,01 Gew.-% und der des Schwefels zu 1,40 Gew.-% sowie das molare Verhältnis von Schwefel zu Nickel zu 0,28 ermittelt.

### II. Oligomerisierungen

Als Einsatzstoff für die Oligomerisierung von Butenen diente "Raffinat II" folgender Zusammensetzung:

| | |
|---|---|
| 29,8 Gew.-% | 1-Buten |
| 31,7 Gew.-% | trans-2-Buten |
| 17,8 Gew.-% | cis-2-Buten |
| 2,1 Gew.-% | Iso-Buten |
| 15,3 Gew.-% | n-Butan |
| 3,1 Gew.-% | Iso-Butan |
| 0,2 Gew.-% | Iso-Pentan |

Die Umsetzung erfolgte kontinuierlich in einem thermostatisierten Rohrreaktor, dessen Reaktionsrohr eine lichte Weite von 30 mm aufwies. Der gegenüber dem Eigendruck des Raffinats II erhöhte Reaktionsdruck wurde mittels einer vorgeschalteten Reaktoreinsatzpumpe erzeugt und mittels üblicher Druckhaltevorrichtungen nach dem Reaktor reguliert. Im Reaktionsrohr wurden über 165 ml des Katalysators zunächst 24 Stunden lang bei 160°C stündlich 1500 Nl trockener Stickstoff geleitet. Anschließend ließ man das Reaktionsrohr mit dem Katalysator unter Stickstoff auf 20°C abkühlen. Danach wurde bei einem Raffinat II-Druck von 20 bar die Temperatur in 24 Stunden auf 80°C erhöht und die Umsetzung bei diesen Bedingungen und einer Katalysatorbelastung von 3,35 kg Raffinat II pro Liter Katalysator und Stunde durchgeführt. Der Reaktionsaustrag wurde isoliert und der Anteil der Isomeren mit 8 Kohlenstoffatomen ("C₈-Selektivität") sowie der ISO-Index der C₈-Fraktion mittels Gaschromatografie bestimmt. Tabelle 3 zeigt die Ergebnisse.

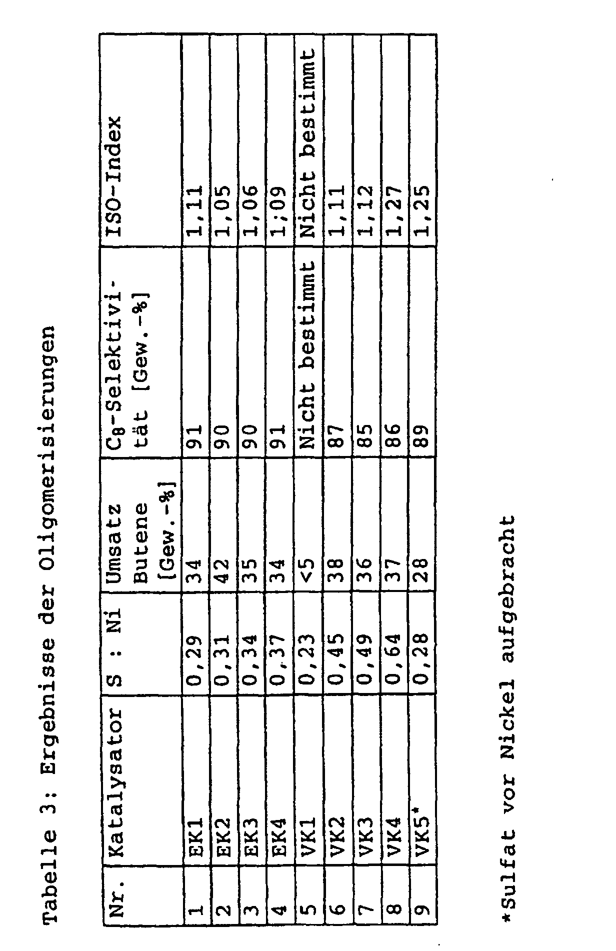

## Patentansprüche

1. Verfahren zur Herstellung eines Oligomerisierungskatalysators für Olefine mit 4 bis 6 Kohlenstoffatomen, bei dem man Aluminiumoxid mit einer Nickelverbindung und einer Schwefelverbindung beaufschlagt, und zwar gleichzeitig oder zuerst mit der Nickelverbindung und danach mit der Schwefelverbindung, und den so erhaltenen Katalysator anschließend trocknet und calciniert, **dadurch gekennzeichnet, dass** man auf diese Weise im fertigen Katalysator ein molares Verhältnis von Schwefel zu Nickel von 0,25 : 1 bis 0,38 : 1 einstellt.

2. Verfahren zur Herstellung eines Oligomerisierungskatalysators für Olefine mit 4 bis 6 Kohlenstoffatomen, bei dem man Aluminiumoxid mit einer Nickelverbindung und einer Schwefelverbindung gleichzeitig beaufschlagt und den so erhaltenen Katalysator anschließend trocknet und calciniert, **dadurch gekennzeichnet, dass** man auf diese Weise im fertigen Katalysator ein molares Verhältnis von Schwefel zu Nickel von 0,25 : 1 bis 0,38 : 1 einstellt.

3. Verfahren zur Herstellung eines Oligomerisierungskatalysators für Olefine mit 4 bis 6 Kohlenstoffatomen, bei dem man Aluminiumoxid zuerst mit einer Nickelverbindung und danach mit einer Schwefelverbindung beaufschlagt und den so erhaltenen Katalysator anschließend trocknet und calciniert, **dadurch gekennzeichnet, dass** man auf diese Weise im fertigen Katalysator ein molares Verhältnis von Schwefel zu Nickel von 0,25 : 1 bis 0,38 : 1 einstellt.

4. Verfahren nach den Ansprüchen 1 bis 3, **dadurch gekennzeichnet, dass** man im fertigen Katalysator ein molares Verhältnis von Schwefel zu Nickel von 0,28 : 1 bis 0,35 : 1 einstellt.

5. Verfahren nach den Ansprüchen 1 bis 4, **dadurch gekennzeichnet, dass** man als Aluminiumoxid γ-Aluminiumoxid oder η-Aluminiumoxid oder deren Gemische verwendet.

6. Verfahren nach Anspruch 5, **dadurch gekennzeichnet, dass** man als Aluminiumoxid γ-Aluminiumoxid verwendet.

7. Oligomerisierungskatalysator, erhältlich nach einem Verfahren gemäß den Ansprüchen 1 bis 6.

8. Verfahren zur Herstellung von Oligomeren von Olefinen mit 4 bis 6 Kohlenstoffatomen oder von Gemischen dieser Olefine bei Temperaturen von 20 bis 280°C und einem Druck von 10 bis 300 bar, **dadurch gekennzeichnet, dass** man es in Gegenwart eines Katalysators gemäß Anspruch 7 durchführt.

9. Verfahren nach Anspruch 8, **dadurch gekennzeichnet, dass** man als Olefine solche mit 4 Kohlenstoffatomen einsetzt.

10. Verfahren nach Anspruch 9, **dadurch gekennzeichnet, dass** man ein Gemisch von 1-Buten und/oder 2-Buten und Butan enthaltenden Kohlenwasserstoffströmen, die im Wesentlichen von Isobuten frei sind, einsetzt.

## Claims

1. A process for producing an oligomerization catalyst for olefins having from 4 to 6 carbon atoms, in which aluminum oxide is treated with a nickel compound and a sulfur compound, either simultaneously or firstly with the nickel compound and then with the sulfur compound, and the resulting catalyst is subsequently dried and calcined, wherein a molar ratio of sulfur to nickel in the finished catalyst of from 0.25:1 to 0.38:1 is set in this way.

2. A process for producing an oligomerization catalyst for olefins having from 4 to 6 carbon atoms, in which aluminum oxide is treated simultaneously with a nickel compound and a sulfur compound and the resulting catalyst is subsequently dried and calcined, wherein a molar ratio of sulfur to nickel in the finished catalyst of from 0.25:1 to 0.38:1 is set in this way.

3. A process for producing an oligomerization catalyst for olefins having from 4 to 6 carbon atoms, in which aluminum oxide is treated firstly with a nickel compound and then with a sulfur compound and the resulting catalyst is subsequently dried and calcined, wherein a molar ratio of sulfur to nickel in the finished catalyst of from 0.25:1 to 0.38:1 is set in this way.

4. A process as claimed in any of claims 1 to 3, wherein a molar ratio of sulfur to nickel in the finished catalyst of from 0.28:1 to 0.35:1 is set.

5. A process as claimed in any of claims 1 to 4, wherein the aluminum oxide used is γ-aluminum oxide or η-aluminum oxide or a mixture thereof.

6. A process as claimed in claim 5, wherein the aluminum oxide used is γ-aluminum oxide.

7. An oligomerization catalyst obtainable by a process as claimed in any of claims 1 to 6.

8. A process for preparing oligomers of olefins having from 4 to 6 carbon atoms or of mixtures of these olefins at from 20 to 280°C and a pressure of from 10 to 300 bar in the presence of a catalyst as claimed in claim 7.

9. A process as claimed in claim 8, wherein the olefins used have 4 carbon atoms.

10. A process as claimed in claim 9, wherein hydrocarbon streams which comprise a mixture of 1-butene and/or 2-butene and butane and are essentially free of isobutene are used.

## Revendications

1. Procédé de préparation d'un catalyseur d'oligomérisation d'oléfines ayant de 4 à 6 atomes de carbone, dans lequel on traite un oxyde d'aluminium avec un composé du nickel et un composé du soufre, simultanément, ou en commençant avec le composé du nickel et en poursuivant avec le composé du soufre, puis on sèche et calcine le catalyseur ainsi obtenu, **caractérisé en ce qu'**on ajuste de cette manière dans le catalyseur fini un rapport en moles du soufre au nickel de 0,25:1 à 0,38:1.

2. Procédé de préparation d'un catalyseur d'oligomérisation d'oléfines ayant de 4 à 6 atomes de carbone, dans lequel on traite un oxyde d'aluminium simultanément avec un composé du nickel et un composé du soufre, puis on sèche et calcine le catalyseur ainsi obtenu, **caractérisé en ce qu'**on ajuste de cette manière dans le catalyseur fini un rapport en moles du soufre au nickel de 0,25:1 à 0,38:1.

3. Procédé de préparation d'un catalyseur d'oligomérisation d'oléfines ayant de 4 à 6 atomes de carbone, dans lequel on traite un oxyde d'aluminium d'abord avec un composé du nickel, puis avec un composé du soufre, puis on sèche et calcine le catalyseur ainsi obtenu, **caractérisé en ce qu'**on ajuste de cette manière dans le catalyseur fini un rapport en moles du soufre au nickel de 0,25:1 à 0,38:1.

4. Procédé selon les revendications 1 à 3, **caractérisé en ce qu'**on ajuste dans le catalyseur fini un rapport en moles du soufre au nickel de 0,28:1 à 0,35:1.

5. Procédé selon les revendications 1 à 4, **caractérisé en ce qu'**on utilise en tant qu'oxyde d'aluminium de l'oxyde d'aluminium γ ou de l'oxyde d'aluminium η ou leurs mélanges.

6. Procédé selon la revendication 5, **caractérisé en ce qu'**on utilise en tant qu'oxyde d'aluminium de l'oxyde d'aluminium γ.

7. Catalyseur d'oligomérisation pouvant être obtenu par un procédé selon les revendications 1 à 6.

8. Procédé de préparation d'oligomères d'oléfines ayant de 4 à 6 atomes de carbone ou de mélange de ces oléfines à des températures de 20 à 280°C et sous une pression de 10 à 300 bar, **caractérisé en ce qu'**on le met en oeuvre en présence d'un catalyseur selon la revendication 7.

9. Procédé selon la revendication 8, **caractérisé en ce qu'**on utilise en tant qu'oléfines des oléfines ayant 4 atomes de carbone.

10. Procédé selon la revendication 9, **caractérisé en ce qu'**on utilise un mélange de courants d'hydrocarbures contenant du 1-butène et/ou du 2-butène et du butane, qui pour l'essentiel sont exempts d'isobutène.
